(19) Europäisches Patentamt
European Patent Office
Office européen des brevets

(11) **EP 1 673 590 B1**

(12) **FASCICULE DE BREVET EUROPEEN**

(45) Date de publication et mention
de la délivrance du brevet:
**06.01.2010 Bulletin 2010/01**

(21) Numéro de dépôt: **04805736.8**

(22) Date de dépôt: **07.10.2004**

(51) Int Cl.:
*A43D 1/02* *(2006.01)*     *A61B 5/103* *(2006.01)*
*A63B 71/06* *(2006.01)*     *G01C 22/00* *(2006.01)*
*G01P 3/00* *(2006.01)*       *G01P 3/50* *(2006.01)*
*G01P 15/00* *(2006.01)*

(86) Numéro de dépôt international:
**PCT/FR2004/050493**

(87) Numéro de publication internationale:
**WO 2005/034751 (21.04.2005 Gazette 2005/16)**

(54) **DISPOSITIF DE CONTROLE DE FOULEE**

SCHRITTÜBERWACHUNGS-EINRICHTUNG

STRIDE-MONITORING DEVICE

(84) Etats contractants désignés:
**AT BE BG CH CY CZ DE DK EE ES FI FR GB GR
HU IE IT LI LU MC NL PL PT RO SE SI SK TR**

(30) Priorité: **10.10.2003 FR 0311883**

(43) Date de publication de la demande:
**28.06.2006 Bulletin 2006/26**

(73) Titulaire: **COMMISSARIAT A L'ENERGIE
ATOMIQUE
75015 Paris (FR)**

(72) Inventeurs:
• **BOUVIER, Alain
  F-38420 Revel (FR)**
• **BLANPAIN, Roland
  F-38380 Entre-Deux-Guiers (FR)**

(74) Mandataire: **Poulin, Gérard et al
Brevalex
3, rue du Docteur Lancereaux
75008 Paris (FR)**

(56) Documents cités:
**US-A- 5 807 283     US-A- 6 122 960**

**Description**

**DOMAINE TECHNIQUE**

La présente invention concerne un dispositif de contrôle (« monitoring ») de la foulée d'un marcheur ou d'un coureur.

Elle trouve des applications notamment dans le domaine du sport et dans le domaine médical.

**ETAT DE LA TECHNIQUE ANTERIEURE**

On connaît déjà des dispositifs qui sont implantés dans les paires de chaussures et destinés à contrôler certains paramètres. Un dispositif de ce genre peut comprendre une masse magnétique, placée dans l'une des chaussures, et un moyen de mesure placé dans l'autre chaussure.

On se reportera en particulier aux documents suivants :

[1] DE 29701308 A
[2] CA 1193436 A.

Le document [1] décrit un dispositif électronique de mesure du mouvement d'un pied dans une chaussure, par exemple au moyen de transducteurs à effet Hall et d'aimants fixés à la semelle de cette chaussure.

Le document [2] décrit un dispositif destiné à avertir un enfant lorsqu'il se trompe de pied en mettant ses chaussures, à l'aide d'un aimant placé dans l'une des chaussures et de moyens électriques et magnétiques placés dans l'autre chaussure.

Remarquons que ces documents ne divulguent pas l'utilisation d'un accéléromètre ou d'un magnétomètre.

US 5 807 283 décrit un dispositif de surveillance d'activité. US 6 122 960 décrit un procédé et un système de mesure du mouvement d'objets.

**EXPOSÉ DE L'INVENTION**

La présente invention a pour but de résoudre le problème de l'obtention d'informations sur la foulée d'un marcheur ou d'un coureur.

Elle vise à mesurer des paramètres caractéristiques de la foulée et éventuellement d'autres paramètres qui sont complémentaires des précédents (notamment les mouvements du pied), à l'aide de moyens appropriées, contenus dans les chaussures de la personne dont on contrôle la foulée.

L'invention propose d'utiliser au moins un magnétomètre pour faire des mesures de champ magnétique et au moins un accéléromètre pour faire des mesures d'accélération au cours du déplacement du dispositif afin de calculer la position dans l'espace de ce dispositif.

De façon précise, la présente invention a pour objet un dispositif de contrôle de foulée conformément à la revendication 1.

Notons que l'insertion des composants du dispositif dans la paire de chaussures permet de ne pas gêner la personne qui porte ces chaussures et conduit à un dispositif discret.

Selon un mode de réalisation préféré du dispositif objet de l'invention, chacune des première et deuxième chaussures comprend au moins une masse magnétique, des moyens de mesure, pour effectuer au moins une mesure physique, et des moyens électroniques de traitement de cette mesure physique, les moyens de mesure comprenant au moins un accéléromètres et au moins un magnétomètre qui sont aptes à fournir des signaux dont le traitement permet de détermine des paramètres de la foulée.

De préférence, la masse magnétique comprend au moins un aimant permanent.

Les moyens de mesure peuvent comprendre une pluralité d'accéléromètres.

De même, les moyens de mesure peuvent comprendre une pluralité de magnétomètres.

De préférence, les moyens électroniques de traitement sont munis de moyens de transmission d'un signal fourni par ces moyens électroniques de traitement.

Selon un mode de réalisation particulier du dispositif objet de l'invention, ce dispositif comprend en outre des moyens portables prévus pour recevoir le signal transmis par les moyens de transmission et afficher des données représentatives de ce signal.

De préférence, ces moyens portables comprennent :

- des moyens de réception de données,
- des moyens électroniques de traitement de ces données, ces moyens électroniques de traitement de données étant munis d'une mémoire,
- des moyens d'introduction de commande, et
- des moyens d'affichage.

Selon un mode de réalisation préféré de l'invention, la mémoire contient :

- une séquence de calibration du signal transmis par les moyens de transmission, en fonction de la longueur de la foulée et de paramètres intrinsèques des chaussures,
- un algorithme d'estimation de la longueur de la foulée,
- un algorithme de calibrage du signal transmis par les moyens de transmission, en fonction de paramètres fournis par un utilisateur, et
- un algorithme d'estimation de la vitesse de la foulée.

De préférence, la séquence de calibration est prévue pour déterminer, d'une part, une loi mathématique de calibration au moyen d'une régression polynomiale et, d'autre part, une correspondance directe entre le signal mesuré et la longueur de la foulée, pour des chaussures et un individu donnés.

De préférence, l'algorithme d'estimation de la longueur de la foulée utilise la mesure de la variation du champ magnétique résultant du mouvement de la masse magnétique.

## BRÈVE DESCRIPTION DES DESSINS

La présente invention sera mieux comprise à la lecture de la description d'exemples de réalisation donnés ci-après, à titre purement indicatif et nullement limitatif, en faisant référence aux dessins annexés sur lesquels :

- la figure 1 est une vue schématique d'une partie d'un mode de réalisation particulier du dispositif objet de l'invention, qui est contenue dans l'une des chaussures d'une paire de chaussures,
- la figure 2 est une vue schématique d'une autre partie de ce dispositif, qui est contenue dans l'autre chaussure de la paire,
- la figure 3 est une vue schématique de moyens de contrôle et de commande que comporte ce dispositif,
- la figure 4 est un diagramme de signaux qui sont reçus par des composants de ce dispositif, et
- la figure 5 illustre schématiquement un dispositif de capture de mouvements.

## EXPOSÉ DÉTAILLÉ DE MODES DE RÉALISATION PARTICULIERS

On décrit dans ce qui suit des exemples du dispositif objet de l'invention, dans lesquels on utilise le fait que, dans une foulée, à tout instant l'un des pieds possède un point d'appui fixe par rapport au sol tandis que l'autre pied est mobile, à une distance variable du pied possèdant le point d'appui fixe par rapport au sol, et l'on mesure cette distance pour calculer, par sommation, la distance parcourue.

Ces exemples mettent en oeuvre l'association conjuguée et le traitement des signaux d'un ou de plusieurs magnétomètres (de préférence un ou plusieurs micro-magnétomètres) et d'un ou de plusieurs accéléromètres, qui sont contenus dans l'une des chaussures d'une paire de chaussures, et une masse magnétique, qui est contenue dans l'autre chaussure de la paire, pour mesurer principalement des distances et des vitesses.

On utilise ainsi au moins un magnétomètre, de préférence au moins un micro-magnétomètre, qui mesure le champ magnétique produit par une masse magnétique mobile, pour faire une mesure dynamique de la distance entre les chaussures grâce à la mesure de la réponse du signal du magnétomètre. Cette mesure utilise la forme du signal et l'amplitude du champ magnétique qui est alternatif et que l'on mesure au rythme de la foulée.

La calibration temporelle de cette mesure dynamique se fait grâce au signal qui est fourni par l'accéléromètre, en particulier au moment du choc du pied sur le sol et permet de déterminer les instants où le signal du micro-magnétomètre doit être traité.

Cette mesure peut être corrigée ou affinée par l'intégration conjointe de l'accélération mesurée par l'accéléromètre, de la mesure de la vitesse de la masse magnétique mobile puis de la mesure de la distance entre les chaussures.

Cette mesure sera calibrée par une phase d'étalonnage pour établir la loi donnant la longueur de la foulée en fonction de l'amplitude du signal et en fonction du marcheur ou du coureur et des caractéristiques magnétiques de la chaussure contenant la masse magnétique.

Dans une variante du dispositif, on peut faire une mesure différentielle entre, le micro-magnétomètre implanté dans la chaussure et un autre micro-magnétomètre, qui est distant de cette chaussure et qui est par exemple dans un bracelet, pour soustraire le champ magnétique terrestre (mesuré par cet autre micro-magnétomètre) du champ magnétique mesuré par le micro-magnétomètre qui est implanté dans la chaussure, afin d'améliorer le rapport signal/bruit et donc la précision de la mesure.

Dans les exemples donnés, les chaussures sont pourvues de moyens complètement autonomes et portables.

Les paramètres mesurés sont de préférence transmis par radio à des moyens personnels de visualisation et de contrôle qui peuvent être installés sur un bracelet ou sur tout autre élément portable.

On peut réaliser un dispositif conforme à l'invention permettant de mesurer divers paramètres, en particulier le nombre de pas, la longueur de chaque pas, la distance parcourue, le temps de marche ou de course, la vitesse moyenne du marcheur ou du coureur, sa vitesse maximale et son temps de repos.

Ce dispositif peut être programmé pour définir une randonnée-type, notamment par la durée, la vitesse, le rythme, le temps de repos, et mesurer les écarts entre les valeurs effectives et les valeurs prévues pour que l'utilisateur réalise un programme défini.

Ce dispositif peut comporter deux micro-magnétomètres supplémentaires en vue d'enregistrer les directions et/ou la route et/ou le cap, qui sont suivis par l'utilisateur du dispositif.

Tous les paramètres sont transmis aux moyens portables de visualisation (qui peuvent être fixés à une ceinture ou à un bracelet).

En outre, à un dispositif conforme à l'invention on peut adjoindre une montre et/ou un altimètre et/ou un moyen de mesure de température et/ou un micro-magnétomètre (pour mesurer le champ magnétique terrestre, comme on l'a déjà vu plus haut) et/ou un moyen de mesure du rythme cardiaque au niveau du poignet.

Un indice énergétique du porteur du dispositif peut être également calculé :

IE(t)=K.(a.Dm+b.Dd+c.Dh)(t), avec :

IE(t) : indice énergétique sur une durée t,

a, b, c : coefficients de pondération, tenant compte, en particulier, du poids de la personne qui porte le dispositif,

Dm : valeur calculée en fonction de la distance parcourue en montant, avec corrélation des informations fournies par l'altimètre,

Dd : valeur calculée en fonction de la distance parcourue en descendant, avec corrélation des informations fournies par l'altimètre,

Dh : valeur calculée en fonction de la distance parcourue en phase horizontale, avec corrélation des informations fournies par l'altimètre,

K : coefficient global, tenant compte des unités, de la nature et de la difficulté du terrain.

On peut définir un indice de la puissance fournie pendant le temps t par la formule suivante :

$$IP(t) = IE(t)/t.$$

Pour des applications sportives de haut niveau ou des applications médicales, on peut réaliser un dispositif conforme à l'invention, ayant une grande sensibilité et utilisant, pour ce faire, plusieurs accéléromètres, plusieurs micromagnétomètres et des algorithmes de traitement qui sont élaborés pour calculer, avec une grande précision, les mouvements du pied dans l'espace et ses différentes orientations, dans une phase de temps déterminée.

Un dispositif conforme à l'invention est installé dans une paire de chaussures. L'une des chaussures peut être simplement pourvue d'une masse magnétique constituée par un aimant, de préférence un aimant permanent.

L'autre chaussure peut simplement comporter au moins un accéléromètre, au moins un magnétomètre, des moyens électroniques de traitement des signaux fournis par ces derniers et une source d'énergie électrique pour alimenter l'accéléromètre, le magnétomètre et les moyens électroniques de traitement.

Le dispositif peut comporter en outre (mais ce n'est pas indispensable) des moyens de visualisation, de contrôle et de commande (« control ») que l'on peut alors placer sur un bracelet ou une ceinture.

Cependant, pour des raisons de fabrication industrielle, de quantités fabriquées, de symétrie et d'homogénéité, les deux chaussures reçoivent de préférence le même équipement.

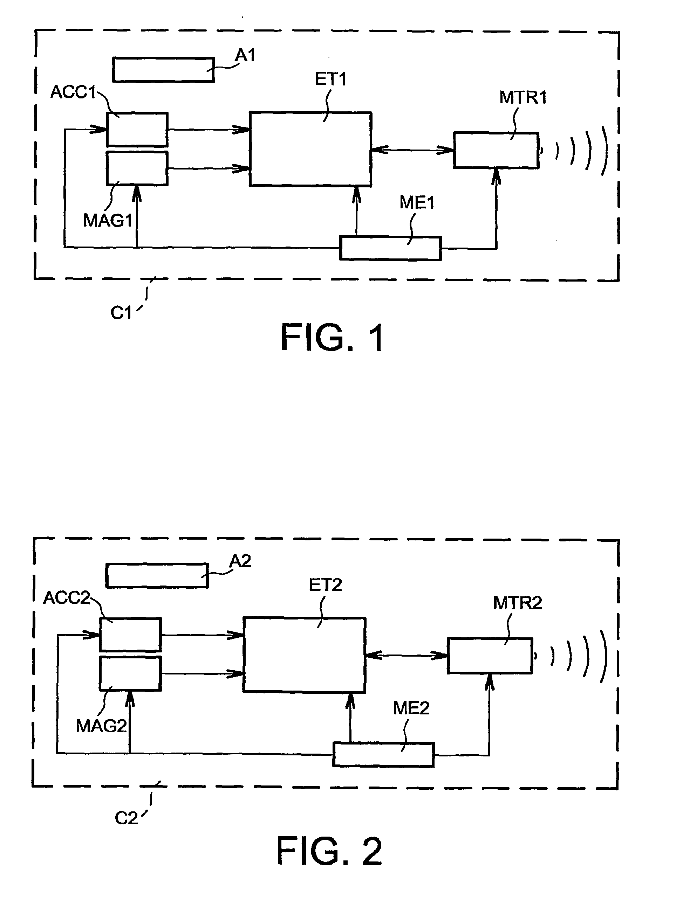

Ceci est schématiquement illustré par l'exemple des figures 1 et 2 où un dispositif conforme à l'invention est installé dans une paire de chaussures qui ont les références C1 et C2 sur les figures 1 et 2, la chaussure C1 correspondant par exemple au pied droit et la chaussure C2 au pied gauche. Remarquons que ces chaussures sont dans le champ magnétique terrestre Bt.

On peut par exemple installer les divers composants du dispositif dans les semelles des chaussures C1 et C2.

La chaussure C1 (respectivement C2) comprend :

- un accéléromètre ACC1 (respectivement ACC2) ou plusieurs accéléromètres si cela est nécessaire,
- un magnétomètre MAG1 (respectivement MAG2) ou plusieurs magnétomètres si cela est nécessaire,
- un aimant permanent A1 (respectivement A2) qui produit un champ magnétique B1 (respectivement B2),
- des moyens électroniques de traitement ET1 (respectivement ET2) pour traiter les signaux fournis par l'accéléromètre et le magnétomètre correspondants,
- un module de transmission MTR1 (respectivement MTR2) qui est muni d'une antenne (non représentée) et prévu pour transmettre les signaux ainsi traités, et
- un module d'alimentation en énergie électrique ME1 (respectivement ME2) qui est destiné à alimenter l'accéléromètre, le magnétomètre, les moyens électroniques et le module de transmission correspondants et qui peut être une pile, un générateur électromécanique ou un accumulateur rechargeable par télé-alimentation.

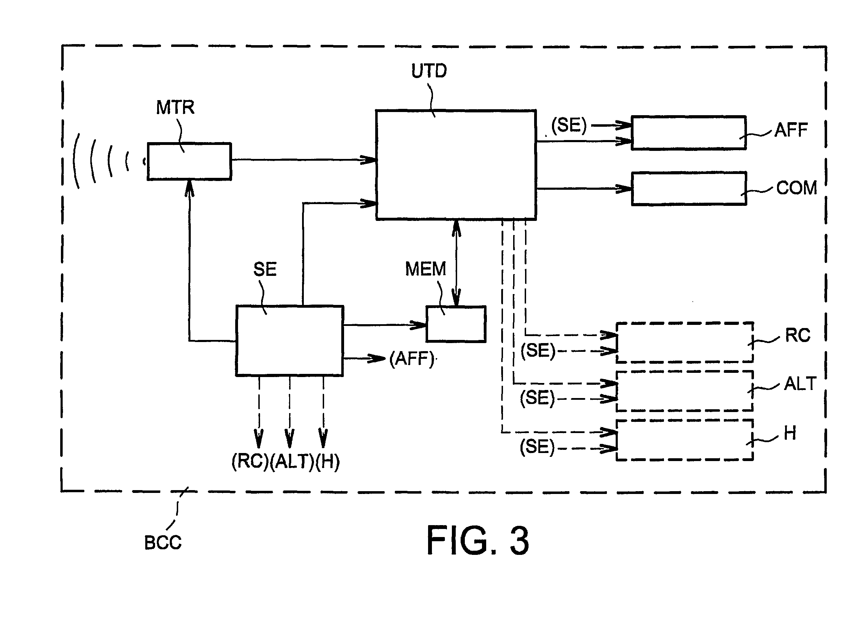

Dans l'exemple considéré, le porteur des chaussures ainsi équipées, possède également, comme on le voit sur la figure 3, un bracelet (ou une ceinture) de contrôle et de commande BCC qui comprend :

- un module MTR de transmission/réception numérique de données, ce module MTR étant muni d'une antenne (non représentée) et permettant la réception des données transmises par les modules MTR1 et MTR2,
- une unité de traitement des données UTD, qui est munie d'une mémoire MEM, d'un clavier de commande COM et de moyens AFF d'affichage des données qui sont reçues par l'intermédiaire du module MTR, ces données étant affichées après avoir été traitées dans l'unité de traitement UTD, et
- une source d'énergie électrique SE, par exemple une pile, pour alimenter en énergie le module MTR, l'unité UTD, la mémoire MEM, les moyens d'affichage AFF (et d'autres organes RC, ALT et H que le bracelet peut éventuellement comporter et dont il sera question par la suite).

Dans la mémoire MEM sont enregistrés par exemple des programmes appelés SEQCAL, FOULEE, NORPARAM et ESTIVITS.

SEQCAL est une séquence de calibration du signal fourni par les accéléromètres et magnétomètres , en fonction de la longueur de la foulée et des paramètres intrinsèques de la chaussure.

SEQCAL calcule la loi mathématique de calibration, à l'aide d'une régression polynomiale ou de tout autre algorithme approprié, et établit une correspondance directe entre le signal de mesure et la longueur de la foulée pour une chaussure et un individu donnés.

Cette correspondance dépend de la masse magnétique propre, de sa répartition dans la chaussure (dans l'exemple considéré, il n'y a qu'un aimant par chaussure mais dans un autre exemple il pourrait y en avoir plusieurs), et de l'orientation du ou des dipôles magnétiques de la masse magnétique.

FOULEE est un algorithme d'estimation de la longueur de la foulée. Cet algorithme est fondé sur le traitement du signal qui est engendré par la variation du champ magnétique que crée la chaussure « magnétique » (elle est appelée ainsi car elle contient un aimant) lorsque cette chaussure est en mouvement.

Le signal varie selon la distance instantanée entre le micro-magnétomètre et la chaussure, la direction de l'aimantation et la répartition de la masse magnétique dans la chaussure.

En fonction de la forme du signal, de son amplitude, de l'instant donné par le ou les accéléromètres pour la prise en compte du signal magnétique de la foulée, et des paramètres de la loi, qui sont déterminés par la phase de calibration, l'unité UTD calcule la longueur de la foulée.

On utilise alors les relations et l'algorithme qui suivent.

Le signal magnétique B(t) mesuré à l'instant t dépend d'un modèle qui est fonction (en première approximation) de la distance instantanée (distance à l'instant t̲) entre la masse magnétique (aimant) de la chaussure et le ou les magnétomètres de l'autre chaussure, selon la relation:

$$\mathbf{B}(t) = \frac{\mu_0}{4\pi}\left(\frac{3(\mathbf{M}.\mathbf{r})\,\mathbf{r}}{r^5} - \frac{\mathbf{M}}{r^3}\right)$$

Dans cette formule, les vecteurs sont indiqués par des caractères gras, $\mu_0$ est la perméabilité du vide ($4\pi \times 10^{-7}$ m.kg.C$^{-2}$), M est le moment magnétique de l'aimant et r est égal à OP où O est le milieu de l'aimant et P le point de mesure.

La signature magnétique complète du passage d'un pied devant l'autre dépend, quant à elle, de la distance minimale entre les deux pieds et de la vitesse V du pied dans la foulée.

La distance minimale entre les deux pieds est déterminée dans l'algorithme précédent (SEQCAL). Donc, par un procédé connu de minimisation des écarts quadratiques et par un filtrage adapté, on peut avoir une estimation correcte de la vitesse V.

La prise en compte des instants d'impact de la chaussure est réalisée par seuillage du ou des accéléromètres, lors du traitement du signal, et permet de déterminer les instants d'impact t1 et t2 et de calculer l'écart δt entre ceux-ci. La longueur L de la foulée est alors facilement calculée : elle est égale au produit de la vitesse V (estimée) par δt.

Il est possible que le signal magnétique soit atténué en fin de foulée et donc que la mesure soit trop imprécise dans cette zone (qui représente quelques % de la course ou de la marche).

Dans ces conditions, avec l'estimation de la vitesse faite lorsque le signal est de bonne qualité, on peut facilement calculer la distance parcourue dans cette zone en multipliant la vitesse estimée par le temps de parcours correspondant à cette zone dont la fin est déterminée par le déclenchement de l'accéléromètre.

De plus, afin d'améliorer la qualité du signal de mesure qui est fourni par les micro-magnétomètres contenus dans les chaussures, cette mesure peut être corrigée en tenant compte de la valeur du champ magnétique terrestre Bt. Pour ce faire, on soustrait de cette mesure la mesure de Bt, qui est faite par le micromagnétomètre fixé au bracelet ou à la ceinture, ce dernier magnétomètre n'étant pas sensible aux aimants des chaussures.

NORPARAM est un algorithme de calibrage des signaux en fonction des paramètres introduits par l'utilisateur sur le clavier du bracelet (ou de la ceinture).

ESTIVITS est un algorithme d'estimation de la vitesse de la foulée. Cet algorithme prend en compte la dérivée du signal émis par le magnétomètre d'une chaussure à la suite de la variation du champ magnétique engendré par l'aimant de l'autre chaussure.

Le bracelet (ou la ceinture) peut être également pourvu :

- d'un capteur de rythme cardiaque RC, constitué par un capteur de pression pour mesure du pouls,
- d'un altimètre numérique ALT dont les données en fonction du temps sont enregistrées dans une mémoire qui est initialisée à chaque course (ou chaque marche) et
- d'une horloge H.

Dans l'unité UTD, on prévoit des algorithmes que l'on associe à ces composants RC, ALT et H et qui permettent de calculer des paramètres secondaires.

On prévoit en particulier un algorithme appelé INDICE pour calculer l'indice énergétique IE et l'indice de puissance IP qui ont été définis plus haut, en fonction des paramètres a, b, c, K.

D'autres algorithmes peuvent être également prévus pour calculer d'autres paramètres tels que la distance parcourue, la vitesse moyenne, la vitesse maximale instantanée, l'énergie totale dépensée par le coureur ou le marcheur, l'énergie instantanée intégrée selon différentes phases, la puissance fournie, l'état des écarts de la course (ou de la marche) en fonction d'un programme préalablement défini.

En outre, un autre algorithme, appelé ORIENTATION, peut être prévu pour calculer, à partir des signaux des accéléromètres et surtout des magnétomètres, qui doivent alors être bi-axes ou tri-axes, les positions exactes et les orientations du pied dans l'espace en cours de foulée.

On reviendra sur un tel traitement à la fin de la présente description.

Si la masse magnétique qu'il est nécessaire de placer dans une chaussure est trop importante, on peut utiliser un électro-aimant à la place d'un aimant permanent pour produire le champ magnétique. De préférence, cet électro-aimant est placé dans la semelle de la chaussure.

L'énergie de l'électro-aimant peut être produite par un générateur, à chaque foulée pendant la course ou la marche, soit lors de l'impact au sol, soit lors du pliage de la semelle. La pile sert alors uniquement à l'alimentation du reste des moyens électroniques.

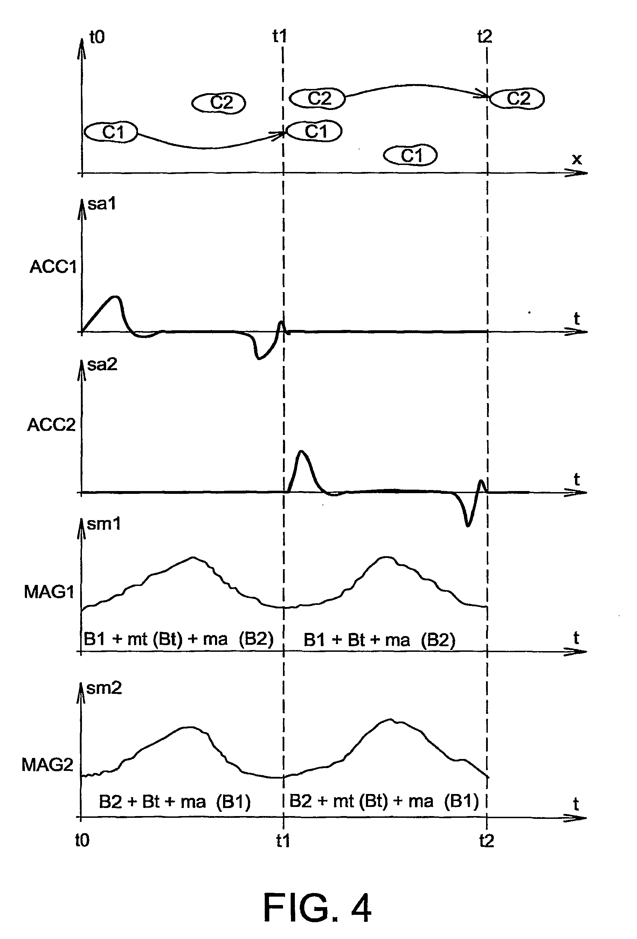

La figure 4 montre le diagramme de signaux sa1, sa2, sm1 et sm2 qui sont respectivement reçus par les accéléromètres ACC1 et ACC2 et par les magnétomètres MAG1 et MAG2, en fonction du temps $\underline{t}$.

Le paramètre x représente la distance parcourue.

A l'instant t0, la chaussure C2 (pied gauche) est à l'arrêt, et la chaussure Cl (pied droit) démarre son mouvement de foulée qui se termine à l'instant t1.

Dans ces conditions, en ce qui concerne les signaux reçus par C1 :

ACC1 enregistre l'accélération puis la décélération de la foulée 1, et
MAG1 mesure un signal s1 qui est la somme de :

- B1 qui est une constante au cours du temps ; elle est connue et peut être facilement soustraite ;
- mt(Bt) qui est une modulation de la mesure du champ magnétique terrestre au cours de la foulée, modulation due aux variations de l'angle que fait MAG1 avec le vecteur Bt ;
- ma(B2) qui est une modulation de l'amplitude du champ B2 durant la foulée, modulation due au passage de MAG1 près de l'aimant A2.

En ce qui concerne les signaux reçus par C2 :

ACC2 ne mesure aucune accélération, C2 étant à l'arrêt, et la vitesse est nulle ;

MAG2 mesure un signal s2 qui est la somme de :

.B2 qui est une constante au cours du temps ; elle est connue et peut être facilement soustraite ;
.Bt qui est constant sur toute cette foulée ;
ma(B1) qui est une modulation de l'amplitude du champ B1 durant la foulée.

A l'instant t1 la foulée 1 est terminée, CI est à l'arrêt et C2 démarre pour s'arrêter à l'instant t2.

On a exactement le même fonctionnement, mais de façon symétrique : les signaux d'indice 1 sont remplacés par les signaux d'indice 2 (et réciproquement). On se reportera à la figure 4.

Considérons maintenant le fonctionnement du dispositif.

Les signaux de MAG1, ACC1, MAG2 et ACC2 sont mesurés sous forme analogique puis convertis sous forme numérique par un convertisseur adapté.

Ces mesures entrent dans le module ET1 (respectivement ET2) de la chaussure C1 (respectivement C2) grâce à une interface (non représentée) et sont, d'une part, stockées dans une mémoire dont la gestion est de type FIFO (premier entré premier sorti) et, d'autre part, transmises directement à l'interface MTR1 (respectivement MTR2) qui se charge de les transmettre sous forme codée, par un procédé de radio-transmission numérique multi-canal.

Les données de la chaussure 1 seront transmises par exemple sur le canal 1 de MTR1, et les données de la chaussure 2 sur le canal 2 de MTR2.

Les capteurs sont échantillonnés selon une fréquence-type de 100 Hz, qui est adaptable.

En réception, le module MTR de l'unité UTD des moyens portables de contrôle-commande reçoit les flots de données et, selon le canal de réception, classe ces données en mode liste dans sa mémoire, selon quatre listes différentes pour MAG1, ACC1, MAG2 et ACC2.

En tête de chaque liste, un indicateur de temps est inséré afin de pouvoir faire le repérage temporel de chacune des valeurs des codeurs correspondants. Le nombre des valeurs numériques des capteurs, entre deux indicateurs temporels des listes enregistrées dans la mémoire, dépend de la fréquence d'échantillonnage retenue. Ce nombre vaut par exemple 100 si cette fréquence vaut 100Hz.

La capacité de mémoire de l'unité UTD est telle qu'elle peut au moins stocker l'ensemble des valeurs des capteurs, multiplié par la fréquence d'échantillonnage, multiplié par le temps maximum de marche (ou de course) à enregistrer (par exemple 24 heures).

L'unité UTD calcule en permanence les paramètres de la marche ou de la course et déclenche pour ce faire les différents algorithmes de calcul enregistrés dans sa mémoire de programmes.

Les données qui en résultent sont affichées de manière cyclique sur les moyens d'affichage AFF, par exemple toutes les 10 secondes, ou sur demande explicite du marcheur ou du coureur.

Les signaux sont enregistrés de manière continue au cours du temps et sont stockés dans la mémoire.

Les algorithmes « FOULEE » et « ESTIVITS » sont exécutés cycliquement afin de calculer :

- la vitesse puis la longueur de la foulée, par l'intégration des signaux des accéléromètres, et
- les valeurs de la vitesse et de la longueur de la foulée par un traitement du signal après soustraction des mesures constantes de Bt, et des bruits de la modulation de la foulée.

Les prises en compte des signaux de MAG1 et MAG2 sont synchronisées avec les déclenchements de détection de début d'accélération de ACC1 et ACC2, afin de ne prendre en compte que des signaux « propres », c'est-à-dire sans perturbation.

L'homme du métier pourrait adapter les exemples qui précèdent au cas où la chaussure C1 est simplement pourvue de l'aimant A1 et la chaussure C2 est simplement pourvue des composants ACC2, MAG2, ET2, MTR2 et ME2.

La présente invention permet à un utilisateur de connaître les paramètres principaux de sa course ou de sa marche. De plus, le dispositif de l'invention peut s'insérer facilement dans des chaussures standard de randonnée ou de jogging, du fait de sa légèreté qui résulte de l'utilisation de technologies intégrées, permettant une réduction de poids et de volume.

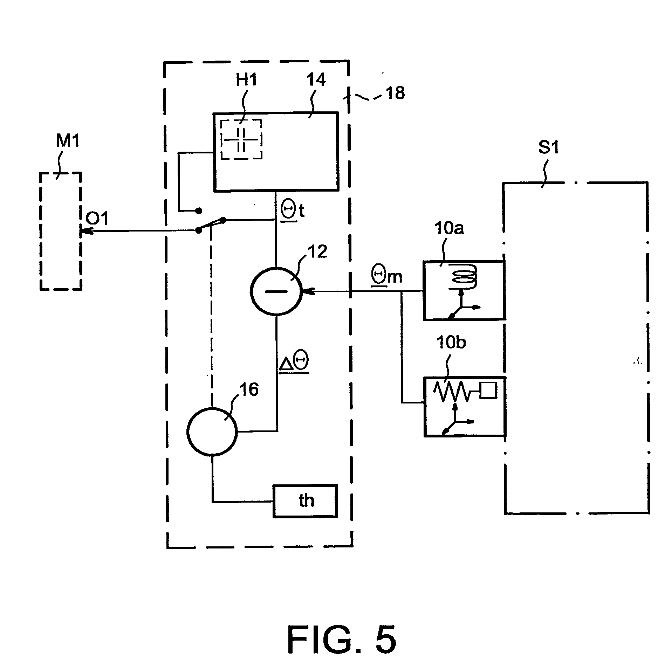

On donne ci-après, en faisant référence à la figure 5, un exemple de technique permettant d'élaborer un algorithme de détermination des orientations du pied dans l'espace.

Les références 10a et 10b indiquent respectivement un accéléromètre et un magnétomètre. Il s'agit de capteurs à trois axes de sensibilité, de type connu, susceptibles de délivrer des données de mesure représentatives de l'orientation, c'est-à-dire d'une position angulaire d'un solide S1. Le solide S1 est indiqué sommairement en trait discontinu. Il s'agit par exemple d'une partie du corps humain dont on veut apprécier les mouvements, une souris informatique, un outil chirurgical, ....

Les mesures des capteurs, notées $\underline{\Theta}_m$, sont des grandeurs scalaires ou vectorielles. Elles sont représentatives, par exemple, d'angles de lacet, de roulis et de tangage $(\varphi,\psi,\theta)$.

Ces mesures sont dirigées vers un comparateur 12. Il s'agit, dans l'exemple illustré, d'un différenciateur. Le comparateur 12 reçoit aussi une ou plusieurs données de test $\underline{\Theta}_t$ délivrées par un calculateur 14. La donnée de test peut être de type vectoriel et exprimer des angles selon plusieurs axes. Le calculateur 14 est utilisé comme moyen générateur de données de test. Les données de test sont représentatives d'une orientation estimée du solide qui peut être aléatoire ou non. Il s'agit, par exemple, de triplets d'angles de lacet, de roulis et de tangage $(\varphi,\psi,\theta)$. Le calculateur peut être localisé sur le solide S1.

Le comparateur délivre une différence $\Delta\Theta$, qui, selon un ou plusieurs axes, représente un écart entre l'orientation réelle, correspondant à la donnée de mesure, et l'orientation estimée correspondant à la donnée de test. Cet écart est utilisable pour affiner l'orientation estimée du capteur, et donc du solide auquel il est fixé.

Toutefois, il est possible de fixer un seuil th en deçà duquel on considère que l'orientation estimée est suffisamment proche de l'orientation mesurée pour être validée. Ceci peut avoir lieu au moyen d'un deuxième comparateur 16 prévu pour comparer la différence $\Delta\Theta$ avec la valeur de seuil th.

Lorsque la différence est inférieure au seuil en valeur absolue la donnée de test $\Theta_t$, c'est-à-dire l'estimation de la position angulaire est dirigée vers une sortie O1.

En revanche, lorsque la différence est supérieure au seuil, elle est dirigée vers le calculateur 14 pour effectuer une nouvelle estimation de la position. Les comparateurs 12 et 16 constituent ainsi avec le calculateur 14 des moyens 18 de modification de l'orientation estimée du solide S1.

La nouvelle estimation peut être aléatoire. Elle peut aussi être affinée selon un calcul de correction par la méthode de descente de gradient d'erreur.

Le deuxième comparateur peut éventuellement être éliminé. Dans ce cas, la valeur estimée est continuellement affinée jusqu'à la saisie d'une nouvelle valeur de mesure.

Le dispositif de la figure 5 comprend des moyens, par exemple une mémoire, pour enregistrer les valeurs estimées successives, validées, en fonction de mesures successives de la position angulaire du solide. La mémoire M1 peut faire partie du calculateur et peut être localisée sur le solide S1. Les valeurs successives permettent de calculer le mouvement de rotation du solide de même que ses vitesses et accélérations angulaires. Pour démarrer la mesure d'une nouvelle orientation du solide, la première donnée de test générée est avantageusement la valeur estimée validée de la position précédente.

La saisie de valeurs de mesure par les capteurs, et l'enregistrement des valeurs estimées dans la mémoire M1 peuvent être cadencés par une horloge H1.

On peut utiliser un nombre quelconque de capteurs, sous réserve que ce nombre soit supérieur au nombre de variables d'angle I à estimer (le nombre de variables d'angle I à estimer est compris entre 1 et 3). Selon la qualité souhaitée de l'estimation, on peut alors utiliser le nombre minimal de capteurs nécessaire ou un nombre de capteurs supérieur au nombre minimal (redondance).

La contribution de chaque capteur peut être pondérée. Il est alors établi un critère de confiance ou poids Cm qui est associé à chaque composante de la mesure $\Theta m$ afin de prendre cette dernière plus ou moins en compte dans l'algorithme de recherche des angles. Le calcul d'un poids Cm est établi selon les règles suivantes :

- a) le poids Cm a une valeur égale à 1 par défaut,
- b) le poids Cm prend la valeur 0 dans le cas où la mesure délivrée est une valeur aberrante (saturation, valeur traduisant un mauvais fonctionnement, etc.),

- c) le poids Cm a une valeur égale à 0 lorsque le niveau de bruit mesuré par le capteur dépasse un certain seuil, une valeur intermédiaire variant linéairement de 0 à 1 pouvant être appliquée pour des valeurs de bruit variant de la valeur du seuil à une valeur de bruit considérée comme négligeable,
- d) la confiance est réduite sur les accéléromètres si l'accélération totale mesurée s'éloigne en norme de la valeur de la pesanteur,
- e) la confiance est réduite sur les magnétomètres si les magnétomètres enregistrent une variation trop importante de leur norme (on peut alors soupçonner la présence d'objet(s) ferromagnétique(s) à proximité du capteur).

En l'absence de pondération, pour une itération effectuée par le calculateur 14, la modification d'un angle de test I est liée à la grandeur $S_I$ telle que :

$$S_I = \Sigma^N_{n=1} \ ( \ \alpha_{In} \ \Delta\Theta_n \ ),$$

où

n est l'indice d'un capteur,
N est le nombre de capteurs,

$\alpha_{In}$ est un paramètre relatif au capteur d'indice n, calculé de façon usuelle par la descente de gradient,
[0115]  $\Delta\Theta_n$ est l'écart entre l'orientation réelle et l'orientation estimée du capteur d'indice n.
L'introduction d'un poids $Cm_n$ relatif au capteur d'indice n modifie alors l'expression de la grandeur $S_I$ comme suit :

$$S_I = \Sigma^N_{n=1} \ Cm_n \ ( \ \alpha_{In} \ \Delta\Theta_n \ )$$
.

De façon générale, les valeurs d'un poids $Cm_n$ peuvent évoluer continûment entre la valeur 1 (confiance totale sur la mesure effectuée par le capteur d'indice n) et la valeur 0 (absence totale de confiance sur la mesure effectuée par le capteur d'indice n, la mesure effectuée par le capteur d'indice n n'est pas prise en compte).
Ainsi, un procédé d'estimation de l'orientation d'un solide peut comprendre les étapes suivantes :

a) la saisie de données de mesure en provenance d'au moins un capteur de position angulaire et l'établissement d'une donnée de test représentative d'une orientation estimée du solide,
b) la confrontation de la donnée de test et la donnée mesurée,
c) l'établissement d'une nouvelle donnée de test représentative d'une nouvelle orientation estimée du solide, corrigée en fonction de la confrontation précédente,
d) la répétition des étapes b) et c).

Les étapes b) et c) peuvent être répétées jusqu'à ce que la confrontation révèle une différence entre la donnée de test et la donnée de mesure inférieure à un seuil déterminé.
Lors de l'étape c), on peut effectuer un calcul de correction selon la méthode dite de descente de gradient d'erreur.
La confrontation des données de test et de la donnée de mesure peut comprendre l'établissement de données de différence entre des données de test successives et la donnée de mesure.
On peut répéter les étapes a) à d) avec des données de mesure successives.

**Revendications**

1. Dispositif de contrôle de foulée, ce dispositif comprenant une paire de chaussures comportant des première et deuxième chaussures, la première chaussure (C1) comprenant au moins une masse magnétique (A1), la deuxième chaussure (C2) comprenant au moins des moyens de mesure, pour effectuer au moins une mesure physique, et des moyens électroniques (ET2) de traitement de cette mesure physique, les moyens de mesure comprenant au moins un magnétomètre (MAG2) qui est apte à fournir des signaux dont le traitement permet de déterminer des paramètres de la foulée, le magnétomètre étant destiné à mesurer le champ magnétique produit par la masse magnétique, pour faire une mesure dynamique de la distance entre les chaussures, ce dispositif étant **caractérisé en ce que** les moyens de mesure comprennent en outre au moins un accéléromètre (ACC2) qui est apte à fournir des signaux dont le traitement permet de déterminer les paramètres de la foulée, l'accéléromètre permettant de connaître les instants d'impact de la deuxième chaussure, qui comprend cet accéléromètre, la prise en compte des instants d'impact servant à la calibration temporelle de ladite mesure dynamique.

2. Dispositif selon la revendication 1, dans lequel chacune des première et deuxième chaussures (C1, C2) comprend au moins une masse magnétique (A1, A2), des moyens de mesure, pour effectuer au moins une mesure physique, et des moyens électroniques (ET1, ET2) de traitement de cette mesure physique, les moyens de mesure comprenant au moins un accéléromètre (ACC1, ACC2) et au moins un magnétomètre (MAG1, MAG2) qui sont aptes à fournir des signaux dont le traitement permet de déterminer des paramètre de la foulée.

**3.** Dispositif selon l'une quelconque des revendications 1 et 2, dans lequel la masse magnétique comprend au moins un aimant permanent (A1, A2).

**4.** Dispositif selon l'une quelconque des revendications 1 à 3, dans lequel les moyens de mesure comprennent une pluralité d'accéléromètres.

**5.** Dispositif selon l'une quelconque des revendications 1 à 4, dans lequel les moyens de mesure comprennent une pluralité de magnétomètres.

**6.** Dispositif selon l'une quelconque des revendications 1 à 5, dans lequel les moyens électroniques de traitement (ET1, ET2) sont munis de moyens (MTR1, MTR2) de transmission d'un signal fourni par ces moyens électroniques de traitement.

**7.** Dispositif selon la revendication 6, comprenant en outre des moyens portables (BCC) prévus pour recevoir le signal transmis par les moyens de transmission et afficher des données représentatives de ce signal.

**8.** Dispositif selon la revendication 7, dans lequel les moyens portables comprennent :

   - des moyens (MTR) de réception de données,
   - des moyens électroniques (UTD) de traitement de ces données, ces moyens électroniques de traitement de données étant munis d'une mémoire (MEM),
   - des moyens d'introduction de commande (COM), et
   - des moyens d'affichage (AFF).

**9.** Dispositif selon la revendication 8, dans lequel la mémoire (MEM) contient :

   - une séquence de calibration du signal transmis par les moyens de transmission (MTR), en fonction de la longueur de la foulée et de paramètres intrinsèques des chaussures,
   - un algorithme d'estimation de la longueur de la foulée,
   - un algorithme de calibrage du signal transmis par les moyens de transmission, en fonction de paramètres fournis par un utilisateur, et
   - un algorithme d'estimation de la vitesse de la foulée.

**10.** Dispositif selon la revendication 9, dans lequel la séquence de calibration est prévue pour déterminer, d'une part, une loi mathématique de calibration au moyen d'une régression polynomiale et, d'autre part, une correspondance directe entre le signal mesuré et la longueur de la foulée, pour des chaussures et un individu donnés.

**11.** Dispositif selon l'une quelconque des revendication 9 et 10, dans lequel l'algorithme d'estimation de la longueur de la foulée utilise la mesure de la variation du champ magnétique résultant du mouvement de la masse magnétique (A1, A2).

**Claims**

**1.** Stride monitoring device, this device comprising a pair of shoes comprising first and second shoes, the first shoe (C1) comprising at least a magnetic mass (A1), the second shoe (C2) comprising at least measurement means to make at least one physical measurement, and electronic means (ET2) for processing of this physical measurement, the measurement means comprising at least one magnetometer (MAG2) which is capable of outputting signals that can be processed to determine stride parameters, the magnetometer being intended for measuring the magnetic field produced by the magnetic mass, to make a dynamic measurement of the distance between the shoes, this device being **characterised in that** the measurement means further comprise at least one accelerometer (ACC2) capable of outputting signals that can be processed to determine the stride parameters, the accelerometer making it possible to know the instants of impact of the second shoe which comprises this accelerometer, the fact of taking the impact instants into account serving to calibrate in time said dynamic measurement.

**2.** Device according to claim 1, in which each of the first and second shoes (C1, C2) comprises at least one magnetic mass (A1, A2), measurement means for making at least one physical measurement, and electronic means (ET1, ET2) for processing this physical measurement, the measurement means comprising at least one accelerometer (ACC1, ACC2) and at least one magnetometer (MAG1, MAG2) capable of outputting signals that can be processed to determine the stride parameters.

**3.** Device according to any one of claims 1 and 2, in which the magnetic mass comprises at least one permanent magnet (A1, A2).

**4.** Device according to any one of claims 1 to 3, in which the measurement means comprise a plurality of accelerometers.

**5.** Device according to any one of claims 1 to 4, in which the measurement means include a plurality of magnetometers.

**6.** Device according to any one of claims 1 to 5, in which the electronic processing means (ET1, ET2) are provided with means (MTR1, MTR2) of transmitting a signal output by these electronic processing means.

7. Device according to claim 6, also comprising portable means (BCC) designed to receive the signal transmitted by the transmission means and to display data representative of this signal.

8. Device according to claim 7, in which the portable means comprise:

> - data reception means (MTR),
> - electronic means (UTD) for processing these data, these electronic data processing means being provided with a memory (MEM),
> - control input means (COM), and
> - display means (AFF).

9. Device according to claim 8, in which the memory (MEM) contains:

> - a sequence to calibrate the signal transmitted by the transmission means (MTR), as a function of the stride length and intrinsic parameters of the shoes,
> - a stride length estimating algorithm,
> - an algorithm to calibrate the signal transmitted by the transmission means as a function of the parameters input by a user, and
> - an algorithm to estimate the stride speed.

10. Device according to claim 9, in which the calibration sequence is designed firstly to determine a mathematical calibration law by means of a polynomial regression, and secondly to determine a direct correspondence between the measured signal and the stride length, for given shoes and a given individual.

11. Device according to any one of claims 9 and 10, in which the stride length estimating algorithm uses the measurement of the variation in the magnetic field resulting from the movement of the magnetic mass (A1, A2).

**Patentansprüche**

1. Vorrichtung zur Kontrolle eines Laufstils, wobei diese Vorrichtung ein Paar Schuhe umfasst, das einen ersten und einen zweiten Schuh umfasst, wobei der erste Schuh (C1) mindestens eine magnetische Masse (A1) umfasst, wobei der zweite Schuh (C2) mindestens Messmittel, um mindestens eine physikalische Messung durchzuführen, und elektronische Mittel (ET2) zur Verarbeitung dieser physikalischen Messung umfasst, wobei die Messmittel mindestens ein Magnetometer (MAG2) umfassen, das geeignet ist, um Signale zu liefern, deren Verarbeitung das Bestimmen von Parametern des Laufstils ermöglicht, wobei das Magnetometer zum Messen des durch die magnetische Masse erzeugten Magnetfelds bestimmt ist, um eine dynamische Messung der Distanz zwischen den Schuhen vorzunehmen, wobei diese Vorrichtung **dadurch gekennzeichnet ist, dass** die Messmittel ferner mindestens einen Beschleunigungsmesser (ACC2) umfassen, der geeignet ist, um Signale zu liefern, deren Verarbeitung das Bestimmen der Parameter des Laufstils ermöglicht, wobei der Beschleunigungsmesser das Erkennen der Zeitpunkte des Aufsetzens des zweiten Schuhs ermöglicht, der diesen Beschleunigungsmesser umfasst, wobei die Berücksichtigung der Aufsetzzeitpunkte der zeitlichen Kalibrierung der dynamischen Messung dient.

2. Vorrichtung nach Anspruch 1, wobei der erste und der zweite Schuh (C1, C2) jeder mindestens eine magnetische Masse (A1, A2), Messmittel zum Durchführen mindestens einer physikalischen Messung und elektronische Mittel (ET1, ET2) zur Verarbeitung dieser physikalischen Messung umfassen, wobei die Messmittel mindestens einen Beschleunigungsmesser (ACC1, ACC2) und mindestens eins Magnetometer (MAG1, MAG2) umfassen, die geeignet sind, um Signale zu liefern, deren Verarbeitung das Bestimmen von Parametern des Laufstils ermöglicht.

3. Vorrichtung nach einem der Ansprüche 1 und 2, wobei die magnetische Masse mindestens einen Permanentmagneten (A1, A2) umfasst.

4. Vorrichtung nach einem der Ansprüche 1 bis 3, wobei die Messmittel mehrere Beschleunigungsmesser umfassen.

5. Vorrichtung nach einem der Ansprüche 1 bis 4, wobei die Messmittel mehrere Magnetometer umfassen.

6. Vorrichtung nach einem der Ansprüche 1 bis 5, wobei die elektronischen Verarbeitungsmittel (ET1, ET2) mit Mitteln (MTR1, MTR2) zur Sendung eines Signals versehen sind, das durch diese elektronischen Verarbeitungsmittel geliefert wird.

7. Vorrichtung nach Anspruch 6, die ferner tragbare Mittel (BCC) umfasst, die zum Empfangen des durch die Sendemittel gesendeten Signals und zum Anzeigen von Daten vorgesehen sind, die für dieses Signal charakteristisch sind.

8. Vorrichtung nach Anspruch 7, wobei die tragbaren Mittel Folgendes umfassen:

> - Mittel (MTR) zum Empfang von Daten,

- elektronische Mittel (UTD) zur Verarbeitung dieser Daten, wobei die elektronischen Datenverarbeitungsmittel mit einem Speicher (MEM) versehen sind,
- Befehlseingabemittel (COM), und
- Anzeigemittel (AFF).

9. Vorrichtung nach Anspruch 8, wobei der Speicher (MEM) Folgendes umfasst:

    - eine Sequenz zur Kalibrierung des durch die Sendemittel (MTR) gesendeten Signals in Abhängigkeit von der Länge des Laufstils und von intrinsischen Parametern der Schuhe,
    - einen Algorithmus zur Schätzung der Länge des Laufstils,
    - einen Algorithmus zur Kalibrierung des durch die Sendermittel gesendeten Signals in Abhängigkeit von durch einen Benutzer gelieferten Parametern, und
    - einen Algorithmus zur Schätzung der Geschwindigkeit des Laufstils.

10. Vorrichtung nach Anspruch 9, wobei die Kalibrierungssequenz vorgesehen ist, um einerseits mittels einer polynomialen Regression ein mathematisches Kalibrierungsgesetz und andererseits eine direkte Zuordnung zwischen dem gemessenen Signal und der Länge des Laufstils für gegebene Schuhe und eine gegebene Person zu bestimmen.

11. Vorrichtung nach einem der Ansprüche 9 und 10, wobei der Algorithmus zur Schätzung der Länge des Laufstils die Messung der Variation des Magnetfeldes verwendet, die sich aus der Bewegung der magnetischen Masse (A1, A2) ergibt.

**FIG. 1**

**FIG. 2**

EP 1 673 590 B1

FIG. 3

FIG. 4

FIG. 5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- DE 29701308 A
- CA 1193436 A
- US 5807283 A
- US 6122960 A